# EUROPEAN PATENT SPECIFICATION

(11) **EP 1 478 773 B1**
(45) Date of publication and mention of the grant of the patent: **19.09.2007**
(21) Application number: 02791197.3
(22) Date of filing: 30.10.2002
(51) Int. Cl.: C12Q 1/68

(54) **METHODS FOR ASSESSING ACUTE MYELOID LEUKEMIA**
METHODEN ZUR FESTSTELLUNG VON AKUTE MYELOISCHEN LEUKÄMIE
METHODES D'EVALUATION DE LA LEUCEMIE MYELOIDE AIGUE

(30) Priority: 30.10.2001 US 340938 P; 30.10.2001 US 338997 P; 30.10.2001 US 340081 P; 30.10.2001 US 341012 P
(43) Date of publication of application: 24.11.2004
(60) Divisional application: 06010179.7
(73) Proprietor: ORTHO-CLINICAL DIAGNOSTICS, INC., Raritan, NJ 08869-0606 (US)
(72) Inventor: RAPONI, Mitch, San Diego, CA 92121 (US)
(74) Representative: Mercer, Christopher Paul
(86) International application number: PCT/US2002/034784
(87) International publication number: WO 2003/038129

(56) References cited:
- WO-A-01/55170
- WO-A-01/88850
- RININGER J A ET AL: "Differential gene expression technologies for identifying surrogate markers of drug efficacy and toxicity" DRUG DISCOVERY TODAY 01 DEC 2000 UNITED KINGDOM, vol. 5, no. 12, 1 December 2000 (2000-12-01), pages 560-568, XP002249959 ISSN: 1359-6446
- BAILEY D S ET AL: "Pharmacogenomics - it's not just pharmacogenetics." CURRENT OPINION IN BIOTECHNOLOGY. ENGLAND DEC 1998, vol. 9, no. 6, December 1998 (1998-12), pages 595-601, XP002249960 ISSN: 0958-1669
- ROWINSKY ERIC K ET AL: "Ras protein farnesyltransferase: A strategic target for anticancer therapeutic development." JOURNAL OF CLINICAL ONCOLOGY, vol. 17, no. 11, November 1999 (1999-11), pages 3631-3652, XP008020349 ISSN: 0732-183X
- BEAUPRE DARRIN M ET AL: "RAS inhibitors in hematologic cancers: Biologic considerations and clinical applications." INVESTIGATIONAL NEW DRUGS, vol. 17, no. 2, 1999, pages 137-143, XP008020350 ISSN: 0167-6997
- BEAUPRE DARRIN M ET AL: "RAS and leukemia: From basic mechanisms to gene-directed therapy." JOURNAL OF CLINICAL ONCOLOGY, vol. 17, no. 3, March 1999 (1999-03), pages 1071-1079, XP008020353 ISSN: 0732-183X

## Description

### BACKGROUND

This invention relates to diagnostics, prognostics, and treatments for leukemia based on the gene expression profiles of leukemia cells.

Some molecules, such as Ras, that are implicated in cancers must be farnesylated by the farnesyl transferase enzyme in order to interact with the inner leaflet of the plasma membrane of the cell and become involved in various signaling pathways. Ras is not the only protein implicated in cancer that has a CAAX box that is prenylated. Farnesyl transferase inhibitors (FTIs) are therapeutic agents that inhibit the covalent attachment of the carbon farnesyl moieties to the C-terminal CAAX motif of various proteins. They have utility in the treatment of cancers and proliferative disorders such as leukemia. Acute myelogenous leukemia (AML) is among the diseases that can most beneficially be addressed with FTIs.

As is true in the case of many treatment regimens, some patients respond to treatment with FTIs and others do not. Prescribing the treatment to a patient who is unlikely to respond to it is not desirable. Thus, it would be useful to know how a patient could be expected to respond to such treatment before a drug is administered so that non-responders would not be unnecessarily treated and so that those with the best chance of benefiting from the drug are properly treated and monitored. Further, of those who respond to treatment, there may be varying degrees of response. Treatment with therapeutics other than FTIs or treatment with therapeutics in addition to FTIs may be beneficial for those patients who would not respond to FTIs or in whom response to FTIs alone is less than desired.

### SUMMARY OF THE INVENTION

The invention is

a method of determining whether a patient with acute myelogenous leukemia (AML) will respond to treatment with a farnesyl transferase inhibitor (FTI) by:
(a) analysing a diseased cell from a bone marrow sample from the patient for a detectable difference in the amount of expression of a gene comprising SEQ ID NO: 846 (3434105F7) following treatment with a FTI relative to an untreated diseased cell baseline;
(b) comparing the detectable difference from step (a) to those obtained from responder and non-responder patients; and
(c) identifying whether the patient has a responder or non-responder expression pattern to determine whether treatment of the patient with a FTI could be indicated.

In another aspect of the invention, there is provided a method of monitoring treatment response in a patient with acute myelogenous leukemia treated with a FTI by:
(a) analysing a diseased cell from a bone marrow sample from the patient for a detectable difference in the amount of expression of a gene comprising SEQ ID NO: 846 (3434105F7) at various periods throughout the course of treatment;
(b) comparing the expression pattern from step (a) to those obtained from responder and non-responder patients; and
(c) identifying whether the patient has a responder or non-responder expression pattern to determine whether treatment of the patient is continued.

A patient may be treated if the gene expression profile shows up regulation of one or more particular genes indicative of FTI responders.

Gene expression profiles indicative of FTI responders are preferably those which show at least a 1.5, 1.7, or 2 fold difference relative to FTI non-responders.

A patient may be treated if the gene expression profile shows down regulation of one or more particular genes indicative of FTI responders

The invention also provides a method wherein step (a) involves analysing the amount of expression of a combination consisting of the genes shown in (i) Tables 1-3 or (ii) Table 1.

Preferably the FTI is a quinilone or quinoline derivative.

Preferably the FTI is (B)-6-[amino(4-chlorophenyl)(1-methyl-1H-imidazol-5-yl)methyl]-4-(3-chlorophenyl)-1-methyl-2(1H)quinolinone).

Articles used in practicing the methods include gene expression profiles or representations of them that are fixed in computer readable media. Other articles include nucleic acid arrays used to determine the gene expression profiles of the invention.

The invention also provides the use of an FTI in the manufacture of a medicament for the treatment of an AML patient, where the patient has been determined to be a responder by a method of the invention. The medicament may comprise a further therapeutic composition. Preferably said further therapeutic composition modulates a MAPK/ERK signalling pathway, TGFβ, WNT or an apoptotic pathway.

The medicament may comprise an FTI and a therapeutic composition selected from the group consisting of tyrosine kinase inhibitors, MEK kinase inhibitors, PI3K kinase inhibitors, MAP kinase inhibitors, apoptosis modulators and combinations thereof.

### BRIEF DESCRIPTION OF THE DRAWINGS

Fig. 1 is an example of a graphical display of gene expression patterns used to analyze the gene expression profiles of this invention.
Fig. 2 is a schematic diagram of the MAPK/ERK pathway.
Fig. 3 is a schematic diagram of the TGFβ and Wnt pathway.
Fig. 4 is a schematic diagram of the apoptotic pathway.

### DETAILED DESCRIPTION

The therapeutic agents referred to in this specification are FTIs. They take on a multitude of forms but share the essential inhibitory function of interfering with or lessening the farnesylation ofproteins implicated in cancer and proliferative diseases. Preferably, the FTIs are those indicated for the treatment of leukemias such as AML. A patient who responds to an FTI is one in whom a reduction of more than 50% of blast cells is seen in bone marrow following treatment with the FTI.

Numerous FTIs are within the scope of the invention and include those described in U.S. Patents: 5,976,851 to Brown et al; 5,972,984 to Anthony et al.; 5,972,966 to deSolms; 5,968,965 to Dinsmore et al.; 5,968,952 to Venet et al.; 6,187,786 to Venet et al.; 6,169,096 to Venet et al.; 6,037,350 to Venet et. al.; 6,177,432 to Angibaud et al.; 5,965,578 to Graham et al.; 5,965,539 to Sebti et al.; 5,958,939 to Afonso et al.; 5,939,557 to Anthony et al.; 5,936,097 to Commercon et al.; 5,891,889 to Anthony et al.; 5,889,053 to Baudin et al.; 5,880,140 to Anthony; 5,872,135 to deSolms; 5,869,682 to deSolms; 5,861,529 to Baudoin; 5,859,0155 to Graham et al.; 5,856,439 to Clerc; 5,856,326 to Anthony et al.; 5,852,010 to Graham et al.; 5,843,941 to Marsters et al.; 5,807,852 to Doll; 5,780,492 to Dinsmore et al.; 5,773,455 to Dong et al.; 5,767,274 to Kim et al.; 5,756,528 to Anthony et al.; 5,750,567 to Baudoin et al.; 5,721,236 to Bishop et al,; 5,700,806 to Doll et al.; 5,661,161 to Anthony et al.; 5,602,098 to Sebti et al.; 5,585,359 to Breslin et al.; 5,578,629 to Ciccarone et al.; 5,534,537 to Ciccarone et al.; 5,532,359 to Marsters et al.; 5,523,430 to Patel et al.; 5,504,212 to de Solms et al.; 5,491,164 to deSolms et al.; 5,420,245 to Brown et al.; and 5,238,922 to Graham et al.. Non-peptidal, so-called "small molecule" therapeutics are preferred. More preferred FTIs are quinolines or quinoline derivatives such as:
7-(3-chlorophenyl)-9-[(4-chlorophenyl)-1H-imidazol-1-ylmethyl]-2,3-dihydro- 1H,5H-benzo[ij]quinolizin-5-one,
7-(3-chlorophenyl)-9-[(4-chlorophenyl)-1H-imidazol-1-ylmethyl]-1,2-dihydro-4H-pyrrolo[3,2,1-ij]quinoline-4-one,
8-[amino(4-chlorophenyl)(1-methyl-1H-imidazol-5-yl),methyl]-6-(3-chlorophen yl)-1,2-dihydro-4H-pyrrolo[3,2,1-ij]quinolin-4-one, and
8-[amino(4-chlorophenyl)(1-methyl-1H-imidazol-5-yl)methyl]-6-(3-chloropheny1)-2,3-dihydro-1H,5H-benzo[ij]quinolizin-5-one.
The most preferred FTI is (B)-6-[amino(4-chlorophenyl)(1-methyl-1H-imidazol-5-yl)methyl]-4-(3-chlorophonyl)-1-methyl-2(1H)-quinolinone).

For treating leukemia with FTIs and other therapeutic agents, the therapeutic agents referred to in this specification are those that have an effect on the biological pathway explicated through the gene expression analysis of leukemic cells subjected to treatment with quinilone-based FTIs.

The mere presence of nucleic acid sequences having the potential to express proteins or peptides ("genes") within the genome is not determinative of whether a protein or peptide is expressed in a given cell. Whether or not a given gene capable of expressing proteins or peptides does so and to what extent such expression occurs, if at all, is determined by a variety of complex factors. Irrespective of difficulties in understanding and assessing these factors, assaying gene expression can provide useful information about the cellular response to a given stimulus such as the introduction of a drug or other therapeutic agent. Relative indications of the degree to which genes are active or inactive can be found in gene expression profiles. The gene expression profiles of this invention are used to identify and treat patients who will likely benefit from a given therapy or exclude patients from a given therapy where the patient likely would experience little or no beneficial response to the drug or therapy.

Preferred methods for establishing gene expression profiles (including those used to arrive at the explication of the relevant biological pathways) include determining the amount ofRNA that is produced by a gene that can code for a protein or peptide. This is accomplished by reverse transcription PCR (RT-PCR), competitive RT-PCR, real time RT-PCR, differential display RT-PCR, Northern Blot analysis and other related tests. While it is possible to conduct these techniques using individual PCR reactions, it is best to amplify copy DNA (cDNA) or copy RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology allows for the measurement of the steady-state mRNA level of thousands of genes simultaneously thereby presenting a powerful tool for identifying the effect ofFTIs on cell biology and the likely effect of treatment based on analysis of such effects. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002 to Linsley, et al.; 6,218,122 to Friend, et al.; 6,218,114 to Peck, et al.; and 6,004,755 to Wang, et al.

Analysis of the expression levels is conducted by comparing such intensities. This is best done by generating a ratio matrix of the expression intensities of genes in a test sample versus those in a control sample. For instance, the gene expression intensities from a tissue that has been treated with a drug can be compared with the expression intensities generated from the same tissue that has not been treated with the drug. A ratio of these expression intensities indicates the fold-change in gene expression between the test and control samples.

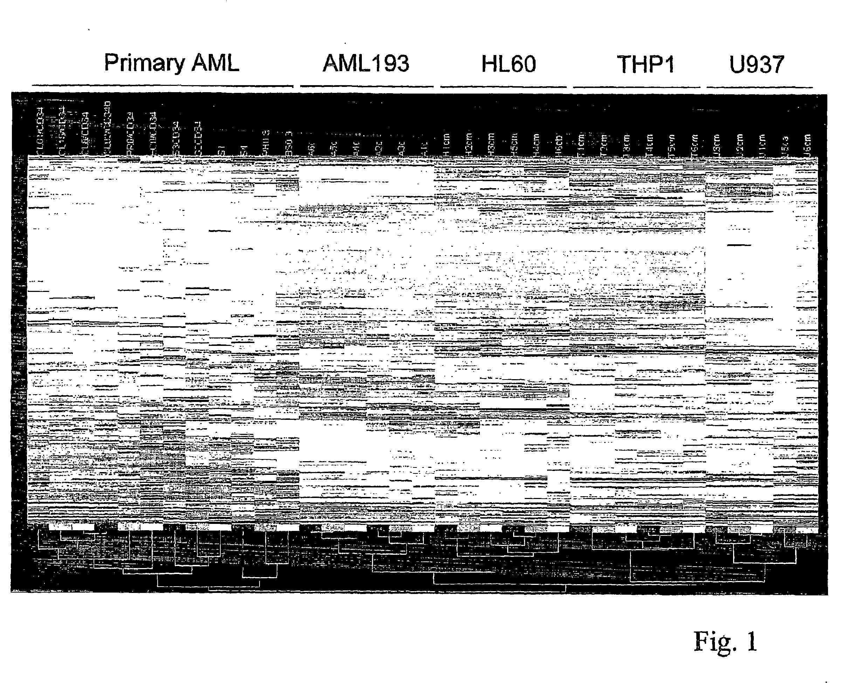

Gene expression profiles can also be displayed in a number of ways. The most common method is to arrange a ratio matrix into a graphical dendogram where columns indicate test samples and rows indicate genes. The data is arranged so genes that have similar expression profiles are proximal to each other (e.g., Fig. 1). The expression ratio for each gene is visualized as a color. For example, a ratio less than one (indicating down-regulation) may appear in the blue portion of the spectrum while a ratio greater than one (indicating up-regulation) may appear as a color in the red portion of the specrtum. Commercially available computer software programs are available to display such data including "OMNIVIZ PRO" software from Batelle and "TREE VIEW" software from Stanford

The genes that are differentially expressed are either up regulated or down regulated in diseased cells following treatment with an FTI. Up regulation and down regulation are relative terms meaning that a detectable difference (beyond the contribution of noise in the system used to measure it) is found in the amount of expression of the genes relative to some baseline. In this case, the baseline is the measured gene expression of the untreated diseased cell. The genes of interest in the treated diseased cells are then either up regulated or down regulated relative to the baseline level using the same measurement method. Preferably, levels of up and down regulation are distinguished based on fold changes of the intensity measurements of hybridized microarray probes. A 1.5 fold difference is preferred for making such distinctions. That is, before a gene is said to be differentially expressed in treated versus untreated diseased cells, the treated cell is found to yield at least 1.5 times more, or 1.5 times less intensity than the untreated cells. A 1.7 fold difference is more preferred and a 2 or more fold difference in gene expression measurement is most preferred. Table 3 lists genes that were commonly modulated across all cell lines and in responder samples.

A portfolio of genes is a set of genes grouped so that information obtained about them provides the basis for making a clinically relevant judgment such as a diagnosis, prognosis, or treatment choice. In this case, the judgments supported by the portfolios involve the treatment ofleukemias with FTIs. Portfolios of gene expression profiles can be comprised of combinations of genes shown in Tables 1-3.

One method of the invention involves comparing gene expression profiles for various genes to determine whether a person is likely to respond to the use of an FTI. Having established the gene expression profiles that distinguish responder from nonresponder, the gene expression profiles of each are fixed in a medium such as a computer readable medium as described below. A patient sample is obtained that contains diseased cells (such as hematopoietic blast cells in the case of AML) is then obtained. Sample RNA is then obtained and amplified from the diseased patient cell and a gene expression profile is obtained, preferably via micro-array, for genes in the appropriate portfolios. The expression profiles of the samples are then compared to those previously determined as responder and non-responder. If the sample expression patterns are consistent with an FTI responder expression pattern then treatment with an FTI could be indicated (in the absence of countervailing medical considerations). If the sample expression patterns are consistent with an FTI non-responder expression pattern then treatment with an FTI would not be indicated. Preferably, consistency of expression patterns is determined based on intensity measurements of micro-array reading as described above.

In similar fashion, gene expression profile analysis can be conducted to monitor treatment response. In one aspect of this method, gene expression analysis as described above is conducted on a patient treated with an FTI at various periods throughout the course of treatment. If the gene expression patterns are consistent with a responder then the patient's therapy is continued. If it is not, then the patient's therapy is altered as with additional therapeutics such as tyrosine kinase inhibitor, changes to the dosage, or elimination ofFTI treatment. Such analysis permits intervention and therapy adjustment prior to detectable clinical indicia or in the face of otherwise ambiguous clinical indicia.

It is possible to attain ambiguous results in which some gene expression profiles are recorded that are in some respects indicative of a responder and in other respects indicative of a non-responder. For example, the profiles may show that three genes are up-regulated consistent with a responder but that another gene is not up-regulated as would ordinarily be the case for a responder. In such a case, statistical algorithms can be applied to determine the probability that the patient will respond or not respond to the drug. Statistical algorithms suitable for this purpose are well known and are available.

Gene expression profiles may be digitally recorded so that they may be compared with gene expression data from patient samples. Alternatively, the profiles can be recorded in different representational format. A graphical recordation is one such format. Fig. 1 shows an example of the graphical display of such a recordation. Clustering algorithms such as those incorporated in "OMNIVIZ" and "TREE VIEW" computer programs mentioned above can best assist in the visualization of such data.

Additional articles used in the invention are nucleic acid arrays (e.g. cDNA or oligonucleotide arrays), as described above, configured to discern the gene expression profiles of the invention.

Using clustering analysis (including the algorithms mentioned above) one can compare the expression levels ofpatient samples to establish regulatory relationships among genes with a certain statistical confidence. A dynamic map was constructed based upon such expression data. Such a genetic network map is useful for drug discovery. For example, once basic genes of interest were identified, a list of potential up-stream regulatory genes was found using such a genetic network map. The genes so identified or their expression products were then analyzed for their use as drug targets. In some embodiments, the regulatory function of the particular genes identified was used to identify therapeutics for use in treating leukemia.

The regulation of transcription, RNA processing and RNA editing are all accomplished by proteins which are coded by their own genes. In addition, DNA sequences can exert long range control over the expression of other genes by positional effects. Therefore, the expression of genes is often regulated by the expression of other genes. Those regulatory genes are called upstream genes, relative to the regulated or downstream genes. In a simple regulatory pathway:
A++>B-->C++>D
where: A, B, C, D are genes
++ up-regulates
- down-regulates
Gene A is an up-stream gene of gene B and B is an up-stream gene of C. One of skill in the art would appreciate that the network is frequently looped and inter-connected. In some instances, the expression of a gene is regulated by its own product as either a positive or negative feedback.

Cluster analysis methods were used to group genes whose expression level is correlated. Methods for cluster analysis are described in detail in Harfigan (1975) Clustering Algorithms, NY, John Wile and Sons, Inc, and Everritt, (1980) Cluster Analysis 2nd. Ed. London Heineman Educational books, Ltd. Path analysis was used to decompose relations among variables and for testing causal models for the genetic networks. Multiple primary targets of a drug in leukemic cells were identified as were drugs/drug classes useful in treating such cells. According to the current invention, drugs are any compounds of any degree of complexity that perturb a biological system.

The biological effect of a drug may be a consequence of drug-mediated changes in the rate of transcription or degradation of one or more species ofRNA, the rate or extent of translation or post-translational processing of one or more polypeptides, the rate or extent of the degradation of one or more proteins, the inhibition or stimulation of the action or activity of one or more proteins, and so forth. In addition to the FTIs that are preferred, the preferred drugs of this invention are those that modulate the MAPK/ERK signaling pathways, TGFβ, WNT or apoptotic pathways. These include, without limitation, tyrosine kinase inhibitors, MEK kinase inhibitors, P 13K kinase inhibitors, MAP kinase inhibitors, apoptosis modulators and combinations thereof. Exemplary drugs that are most preferred among these are the "GLEEVEC" tyrosine kinase inhibitor ofNovartis, U-0126 MAP kinase inhibitor, PD-098059 MAP kinase inhibitor, SB-203580 MAP kinase inhibitor, and antisense, ribozyme, and DNAzyme Bcl-XL anti-apoptotics. Examples of other useful drugs include, without limitation, the calanolides of US Patent 6,306,897; the substituted bicyclics of US Patent 6,284,764; the indolines of US Patent 6,133,305; and the antisense oligonucleotides ofUS Patent 6,271,210.

The invention is further illustrated by the following nonlimiting examples.

### Example 1: Cell culture

The AML-like cell lines HL-60 (promyelocytic) and U-937 (promonocytic) were obtained from the ATCC. AML-193 (monocytic) and THP-1 (monocytic) cells were obtained from the RW Johnson Pharmaceutical Research Center, San Diego. Cells were grown in Roswell Park Memorial Institute medium (RPMI) with 20% Fetal Bovine Serum (FBS). AML-193 was also supplemented with granulocyte-macrophage colony-stimulating factor (GM-CSF) (10 ng/ml), insulin (0.005 mg/ml), and transferrin (0.005 mg/ml).

### Example 2: Toxic Dose Assay

The cells of Example 1 were inoculated into 6-well plates at an initial concentration of 1 x 10⁵ cells/ml. (B)-6-[amino(4-chlorophenyl)(1-methyl-1H-imidazol-5-yl)methyl]-4-(3-chlorophenyl)-1-methyl-2(1H)-quinolinone) was added at concentrations ranging form 0.5 to 500 nM in 3 µl of DMSO directly to the culture medium. Control cells from Example 1 were grown in medium alone or in medium supplemented with vehicle (0.1 % DMSO). Cell numbers were counted at days four and seven in a hemocytometer and cell viability was determined by trypan blue exclusion assay. The IC₅₀ was defined as the dose at which the number of viable cells in the treated sample was 50% of that in the control at day seven. Calculations were made based on duplicate runs of the experiment The IC₅₀ of the four cell lines was calculated after seven days of treatment with the FTI. AML-193 had an IC₅₀ of 134 nM, HL-60 had an IC₅₀ of 24 nM, THP-1 had an IC₅₀ of 19 nM, and U-937 had an IC₅₀ of 44 nM. This indicated that the four AML-like cell lines were sensitive to FTI treatement.

### Example 3: Time Course Assay

Duplicate cultures of the cells of Example 1 were inoculated into 6-well plates at an initial concentration of 1 x 10⁵ cells/ml. (B)-6-[amino(4-chlorophenyl)(1-methyl-1H-imidazol-5-yl)methyl]-4-(3-chlorophenyl)-1-methyl-2(1H)-quinolinone) was supplemented at a concentration of 100 nM in 3 µl of DMSO directly to the culture medium. The concentration of 100 nM was chosen for the subsequent time course experiments to normalize the treatment protocol based, in part, on the results of Example 2. Duplicate control cultures were grown in medium containing 0.1% DMSO. Duplicate cultures were harvested daily for a total of six days. Cells were counted, assayed for viability, and total RNA isolated according to the manufacturer's protocol (Qiagen RNeasy). The analysis showed that cells from different cell lines were effected at different times. RNA was treated with DNase1 (Qiagen DNase1 kit) to remove any residual genomic DNA. Linear amplification of RNA was conducted according to the procedure described in US Patent 5,545,522 to Van Gelder et. al. Aliquots of 5 µg of aRNA were then prepared for hybridization to cDNA arrays.

### Example 4: Bone Marrow Processing

Bone marrow aspirates were obtained from two patients diagnosed with AML who had been treated with FTI. These AML patients were administered 600 mg (B)-6-[amino(4-chlorophenyl)(1-methyl-1H-imidazol-5-yl)methyl]-4-(3-chlorophenyl)-1-methyl-2(1H) quinolinone) twice daily over a 21 day period. Bone marrow aspirates were taken at baseline and once a week for the three weeks of treatment. One of these patients did not respond (RH) while the other responded (BS) to the FTI. Response was determined as a reduction of more than 50% of blast cells in bone marrow aspirates. The aspirates were diluted to 15 ml with PBS and Ficoll-density centrifuged. White blood cells were washed twice with PBS, resuspended in FBS with 10% DMSO and immediately frozen at -80°C. Cells were cryogenically preserved to maintain cell viability. Samples were thawed at 37°C and 10X volume of RPMI with 20% FBS was added drop-wise over a period of 5 min. Cells were centrifuged at 1600 rpm for 10 min and resuspended in 10 ml PBS with 2 mM EDTA and 0.5% BSA. Samples were then passed through a 70 µM filter to remove any cell clumps. Cell viability was determined by Trypan Blue assay. If sample viability was less than 50% a Miltenyi Dead Cell Removal Kit was employed to enrich for the live cell fraction. 2×10⁵ viable cells were then double labeled with CD33-FITC and CD34-PE antibodies (Pharminigen) and FACS analysis was performed. Post (B)-6-[amino(4-chlorophenyl)(1-methyl-1H-imidazol-5-yl)methyl]4-(3-chlorophenyl)-1-methyl-2(1H)-quinolinone)-treated bone marrow samples were enriched for leukemic cells by magnetic bead cell separation using either CD33 or CD34 antibodies (Miltenyi). The extracted cells had RNA extracted as described in Example 3.

### Example 5: Probe Preparation

RNA samples obtained in Examples 3 and 4 were prepared for hybridization to cDNA microarrays according to the following procedure. One to two rounds of linear amplification was performed on total RNA depending on the amount of starting material. Initially, 1-10 µg total RNA was reverse transcribed using the Superscript cDNA transcription kit (Gibco BRL). Ten µl total RNA was first mixed with 1 µl of 0.5 mg/ml T7-oligodT primer, incubated at 70°C for 10 min, and then chilled on ice. Next, 8 µl of 5X first-strand reaction buffer, 0.1M DTT,10 mM dNTPs, and 1 µl Rnase Block were added, and the solution incubated at 42°C for 5 min. One µl Superscript II was then added and the reaction was incubated at 42°C for 2 hr. The reaction was heat deactivted at 70°C for 10 min and 1 µl was removed for PCR. Next, 92 µl Rnase-free water, 30 µl 5X second-strand reaction buffer, 3 µl 10mM dNTP, 4 µl DNA polymerase 1,1 µl *E*. *coli* Rnase H, 1 µl *E*. *coli* DNA ligase were added and the mixture incubated at 16°C for 2 hr. cDNA was linear amplified using the Ampliscribe T7-transcription kit (Epicenter). If required, a second round of RNA amplification was performed by the random hexamer approach. Fluorescently labeled cDNA probes were synthesized by priming aRNA with random hexamers and including Cy3-dCTP in the nucleotide mix. Reactions were purified using a QIAquick PCR purification kit (Qiagen), the volumes of probe normalized using relative fluoresence (Cytofluor), and resuspended in 50 µl of Version 2 hybridization buffer (Amersham Pharmacia Biotech, Pistcataway, NJ) with 50% formamide and human Cot1 DNA (Life Technologies).

### Example 6: Array Hybridization and Analysis

The arrays contained 7452 cDNAs from the IMAGE consortium (Integrated Molecular Analysis of Genome and their Expression: Research Genetics, Huntsville, AL) and Incyte libraries. Micro-arrays were generated as follows and probes hybridized as described in Example 5. cDNAs were printed on amino silane-coated slides (Coming) with a Generation III Micro-array Spotter (Molecular Dynamics). The cDNAs were PCR amplified, purified (Qiagen PCR purification kit), and mixed 1:1 with 10 M NaSCN printing buffer. Prior to hybridization micro-arrays were incubated in isopropanol at room temperature for 10 min. The probes were incubated at 95°C for 2 min, at room temperature for 5 min, and then applied to three replicate slides. Cover slips were sealed onto the slides with DPX (Fluka) and incubated at 42°C overnight. Slides were then washed at 55°C for 5min in 1X SSC/0.2% SDS and O.1X SSC/0.2% SDS, dipped in O.1X SSC and dried before being scanned by a GenIII Array Scanner (Molecular Dynamics). The fluorescence intensity for each spot was analyzed with AUTOGENE software (Biodiscovery, Los Angeles).

The intensity level of each micro-array was normalized so that the 75^{th} percentile of the expression levels was equal across micro-arrays. Clones displaying a coefficient of variance (CV) greater than 50% of the mean were excluded from the analysis. Since background intensity was a maximum of 32 units for all experiments a threshold of 32 was assigned to all clones exhibiting an expression level lower than this. A ratio matrix was then generated based on pair-wise analysis of treated and control samples and Hierarchical clustering was performed using an euclidean metric and average linkage (Omniviz Pro™).

Each sample was hybridized to three identical arrays and the mean signal intensity was compared by scatter-plot analysis. High correlation coefficients were also observed when control samples were compared to treated samples from the same day. This indicated there were no gross changes in gene expression due to treatment with (B)-6-[amino(4-chlorophenyl)(1-methyl-1H-imidazol-5-yl)methyl]-4-(3-chlorophenyl)1-methyl-2(1H)-quinolinone). In addition, the variation between control samples from different days was examined. Cells were mock-treated and RNA was isolated after 1, 2, 3, 4, 5, and 6 days. Following labeling and hybridization the mean intensity of duplicate samples and the coefficient of variance (CV) of each clone (3 spots per clone) were calculated. Data points which displayed a CV of more than 50% were discarded from further analysis.

### Example 7: Differential Gene Expression in Treated Cell Line Samples

Hierarchical clustering was performed on the time-course data sets using the OmniViz Pro™ software (Battelle). Initially, fold-changes of 1.5,1.7, and 2.0 were used as filters for the treated versus control intensity ratios for each day of the time-course. The gene expression profiles of genes modulated beyond these thresholds were analyzed to examine those genes that were commonly modulated between the three data sets and identify gene clusters that shared similar expression profiles. Results are shown in Tables 1-3 below.

Genes analyzed according to this invention are identified in the tables below by reference to Gene ID Numbers (internally generated) and accession numbers in the Genbank database where such genes have been entered in the Genbank database. The attached sequence listing, incorporated herein by reference, shows sequences corresponding to the Gene 1D Number and are named with those Gene ID Numbers. In some cases, the listed sequences are to full length nucleic acid sequences that code for the production of a protein or peptide. One skilled in the art will recognize that identification of full-length sequences is not necessary from an analytical point of view. That is, portions of the sequences or ESTs can be selected according to well-known principles for which probes can be designed to assess gene expression for the corresponding gene. Further, it should be noted that some of the sequences in the listing contain the letter "N" in place of a nucleotide designation. One skilled in the art will recognize that the "N" indicates placement of any nucleotide in that portion of the sequence.

### Example 8 : Identification of Gene Networks

Genes that were regulated in two or more cell lines by at least 1.5-fold in drug treated cell lines (Table 1) were identified as described above. The list of these genes was employed to identify major gene pathways that were being modulated by the most preferred FTI, (B)-6-[amino(4-chlorophenyl)(1-methyl-1H-imidazol-5-yl)methyl]-4-(3-chlorophenyl)-1-methyl-2(1H)-quinolinone). If clones did not perfectly match a known gene annotations from the best BLAST result of the clone sequence were used. Since the level of regulation of these genes varied over the course of treatment of the cell lines, the gene expression profiles from the primary AML tissue that responded to this FTI were determined.

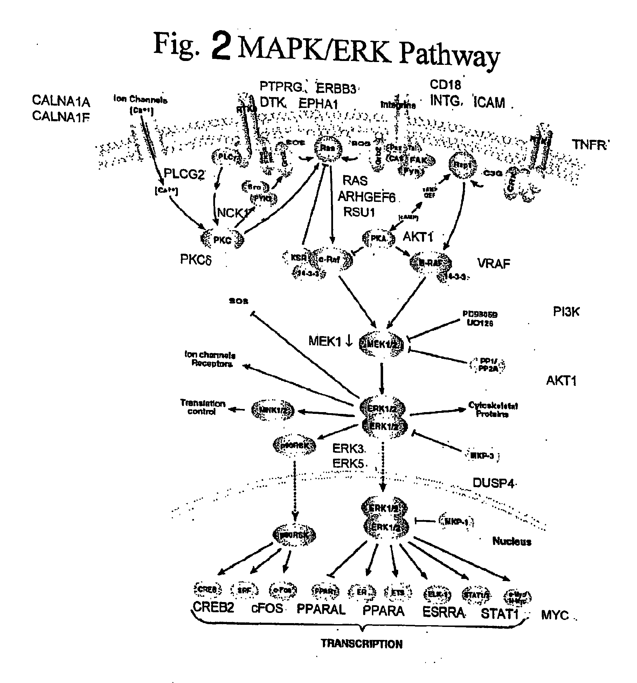

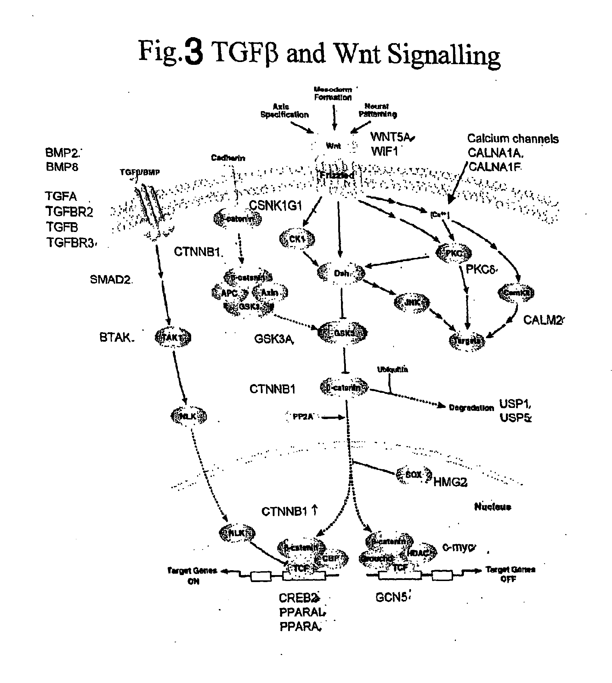

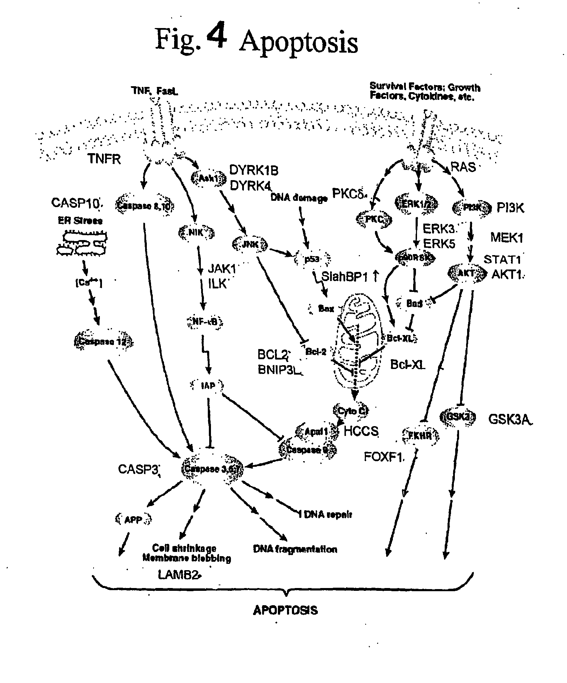

It was found that many genes in the MAPK/ERK (Fig. 2) signaling pathways were being down-regulated and that genes in the TGFβ (Fig. 3) and WNT (Fig. 3) signaling pathways were generally up-regulated, while apoptotic pathways were also activated (Fig.4). This allowed the identification of other gene targets sensitive to treatment with known or novel drug compounds. For example, beneficial treatment can result from FTIs used in conjunction with tyrosine kinase, MEK kinase, PI3K and/or MAP kinase inhibitors to obtain a more potent effect. In addition, given the finding that apoptotic pathways are activated in FTI treated cells, drugs that modulate apoptosis could be expected to have beneficial effect when employed in conjunction with an FTI. Examples of these types of compounds include tyrosine kinase inhibitors (eg Gleevec, Novartis), MAP kinase inhibitors (eg U-0126, PD-098059, SB-203580), and inhibitors of anti-apoptotic genes such as Bcl-XL (eg antisense, ribozymes, DNAzymes).

**Table 1: Genes (by Genbank Accession Number) modulated at least 1.5 fold in 2 or more of the cell lines over the 6 day time course.**

| Gene ID | Accession No. |
|---|---|
| 3434105F7 | AB026898 |
| 3881595H1 | AC000134 |
| AI939481 | AC005155 |
| AA961061 | AC005670 |
| 3918104H1 | AC006023 |
| A1025519 | AC008427 |
| 5543360F8 | AC009220 |
| AA744682 | AC009289 |
| AA932129 | AC009756 |
| AI148008 | AC011473 |
| Y00052 | AC013722 |
| R52476 | AC021078 |
| AA864819 | AC022087 |
| Al815593 | AC022150 |
| Al141943 | AC026448 |
| Al300541 | AC073585 |
| 2515486H1 | AF161372 |
| 1731618H1 | AJ003147 |
| BE222911 | AJ400879 |
| R13802 | AK022901 |
| AI656222 | AL021155 |
| AI553823 | AL022313 |
| AA010251 | AL034397 |
| AA237071 | AL035420 |
| 2445101F6 | AL049824 |
| A1638342 | AL122004 |
| AA779424 | AL136980 |
| H81171 | AL137073 |
| R77754 | AL137790 |
| AI209040 | AL139082 |
| 6421806H1 | AL139396 |
| H61066 | AL161787 |
| R59209 | AL355136 |
| AA486141 | AL355352 |
| AI339252 | AP001630 |
| AW023438 | BC009732 |
| 914979H1 | BC013834 |
| AA455969 | D00015 |
| T64335 | D00017 |
| X02308 | D00596 |
| X67098 | D00596 |
| X01023 | D10493 |
| D10493 | D10493 |
| Y00396 | D10493 |
| X69292 | D10667 |
| AA736561 | D11094 |
| S68252 | D11139 |
| M25315 | D12592 |
| Al186110 | D13118 |
| H84153 | D13639 |
| AA598561 | D14043 |
| D14695 | D14695 |
| 2206642T6 | D16889 |
| Al878943 | D17004 |
| U38846 | D17080 |
| J03801 | D21235 |
| Al762926 | D23660 |
| D25215 | D25215 |
| U41078 | D26512 |
| AA485961 | D30648 |
| AA456408 | D38441 |
| Al311090 | D38616 |
| 1869911H1 | D42084 |
| D43950 | D43950 |
| AW006368 | D44467 |
| U29092 | D45050 |
| D45887 | D45887 |
| W68193 | D49489 |
| 3633286H1 | D63874 |
| N76967 | D66904 |
| AA985407 | D82326 |
| A1962797 | D83260 |
| M59829 | D85730 |
| 3107995H1 | D86322 |
| AA279906 | D86550 |
| AI016874 | D86586 |
| 556963H1 | D86955 |
| AI810687 | D86997 |
| AF045581 | D87462 |
| U41070 | D89078 |
| D90209 | D90209 |
| AA812265 | D90767 |
| 2135769H1 | J02763 |
| 1876511H1 | J03072 |
| J04088 | J04088 |
| Al590075 | J04973 |
| H30357 | J05451 |
| K00558 | K03460 |
| L10413 | L00634 |
| L01087 | L01087 |
| A1027898 | L02426 |
| L06139 | L06139 |
| U28936 | L07914 |
| M86400 | L07955 |
| AA428915 | L08634 |
| AA464627 | L09604 |
| M94859 | L10284 |
| R78541 | L10717 |
| L15189 | L11066 |
| L11284 | L11284 |
| T87908 | L12387 |
| T80827 | L13802 |
| L08044 | L15203 |
| Al278029 | L16862 |
| M60278 | L17032 |
| M60278 | L17032 |
| M65128 | L18980 |
| W49672 | L20861 |
| L22005 | L22005 |
| Al380522 | L23822 |
| AA988469 | L25591 |
| L26336 | L26336 |
| AI074564 | L32866 |
| M63175 | L35233 |
| 2470939H1 | L35848 |
| AA190648 | L36055 |
| Al243166 | L38716 |
| L39833 | L39833 |
| M97347 | L41415 |
| 2005142H1 | L42324 |
| M11723 | L43615 |
| U04045 | L47574 |
| AA284067 | L76938 |
| M13142 | M13142 |
| W68291 | M15395 |
| 5560880H1 | M19483 |
| 3171275H1 | M20199 |
| M22489 | M22489 |
| AA070627 | M22810 |
| H39560 | M24736 |
| M25897 | M25897 |
| M27492 | M27492 |
| AA570304 | M29366 |
| 205581R6 | M29696 |
| M84739 | M32294 |
| M84739 | M32294 |
| M35857 | M32315 |
| 3801801H1 | M33680 |
| M63904 | M63904 |
| AI091579 | M63971 |
| M69215 | M69215 |
| M74782 | M74782 |
| M80254 | M80254 |
| M80647 | M80647 |
| M84526 | M84526 |
| 6805226H1 | M84526 |
| S93414 | M86553 |
| M91556 | M91556 |
| M95678 | M95678 |
| 2017923F6 | M96326 |
| H73054 | NM_000551 |
| AA488324 | NM_001211 |
| N73242 | NM_001274 |
| AF013611 | NM_001335 |
| AW163686 | NM_001524 |
| AI921879 | NM_002287 |
| AI652785 | NM_002333 |
| AI423526 | NM_003332 |
| 3028719F6 | NM_003600 |
| AB010882 | NM_003601 |
| AF030424 | NM_003642 |
| AW194791 | NM_003668 |
| AF075599 | NM_003969 |
| AF031141 | NM_004223 |
| 2676931T6 | NM_004412 |
| AF053304 | NM_004725 |
| AF119815 | NM_004885 |
| A1816398 | NM_004888 |
| 2185556H1 | NM_004917 |
| 3357511H1 | NM_004917 |
| AA047585 | NM_005109 |
| AA448972 | NM_005592 |
| AW629084 | NM_005817 |
| AJ001015 | NM_005854 |
| AJ001016 | NM_005856 |
| U85055 | NM_006480 |
| 3618886F6 | NM_006536 |
| AA906714 | NM_006573 |
| AF060153 | NM_007037 |
| 1467864F6 | NM_012089 |
| AI097079 | NM_012100 |
| AF204944 | NM_012105 |
| W44673 | NM_012428 |
| AI522316 | NM_013386 |
| AI700673 | NM_013439 |
| AA057781 | NM_014172 |
| AI299795 | NM_014251 |
| 1931159F6 | NM_014397 |
| AW630208 | NM_014413 |
| 2821685T6 | NM_014967 |
| 1539060H1 | NM_015343 |
| A1762738 | NM_015449 |
| AA401397 | NM_015596 |
| AW243944 | NM_015596 |
| A1436551 | NM_016141 |
| Al651159 | NM_016440 |
| AA527334 | NM_016625 |
| g922698 | NM_017555 |
| 1693028H1 | NM_017636 |
| 002783H1 | NM_017860 |
| AI368583 | NM_017874 |
| AW170305 | NM_017903 |
| H62827 | NM_018321 |
| M78706 | NM_019020 |
| BE048230 | NM_020216 |
| A1214466 | NM_020334 |
| AA452802 | NM_021196 |
| AI808824 | NM_022082 |
| W77977 | NM_022336 |
| AA535015 | NM_022570 |
| AA861140 | NM_022829 |
| AW078834 | NM_023080 |
| 5122087H1 | NM_024056 |
| AF017182 | NM_024101 |
| AA449040 | NM_024116 |
| 2792728F6 | NM_024902 |
| BE218593 | NM_025230 |
| AA669885 | NM_030763 |
| AI339565 | NM_030908 |
| AF038564 | NM_031483 |
| Al126706 | NM_032038 |
| 1961084H1 | NM_032188 |
| 4609810F6 | NM_032554 |
| AA745592 | NM_032844 |
| AW612141 | NM_033050 |
| Al740538 | NM_033280 |
| U58522 | S51016 |
| M57703 | S63697 |
| AA873257 | S73591 |
| AA521213 | S77359 |
| N77754 | S79873 |
| AA984230 | S80071 |
| R79935 | S81439 |
| T71391 | T71391 |
| AA598776 | U05340 |
| U11053 | U11053 |
| T55353 | U12597 |
| AA456616 | U14970 |
| U18300 | U18300 |
| AF017306 | U20657 |
| AA459663 | U25182 |
| U27699 | U27699 |
| AI884916 | U29171 |
| U29171 | U29171 |
| 1671033F6 | U33429 |
| AA017042 | U40989 |
| Al126520 | U48405 |
| U48807 | U48807 |
| U49395 | U49395 |
| AA186542 | U50078 |
| U51586 | U51586 |
| AW150605 | U54558 |
| AA455800 | U55206 |
| AF029777 | U57316 |
| 119355 | U58913 |
| 319095H1 | U58913 |
| AF027964 | U59911 |
| U60519 | U60519 |
| AI371158 | U65378 |
| U69883 | U69883 |
| H30148 | U73641 |
| R80718 | U75283 |
| U77180 | U77180 |
| U78180 | U78180 |
| U83115 | U83115 |
| AA938905 | U86218 |
| AI401546 | U88844 |
| 2526581H1 | U90904 |
| AA773114 | U95740 |
| AA176596 | U96781 |
| X13274 | V00543 |
| I16618 | V00595 |
| R91899 | X00226 |
| AW519155 | X00318 |
| X87344 | X00369 |
| X02910 | X01394 |
| M15840 | X02532 |
| AA401046 | X02592 |
| X02812 | X02812 |
| X87344 | X03066 |
| X03084 | X03084 |
| M10901 | X03225 |
| X03225 | X03225 |
| R81823 | X03742 |
| X04011 | X04011 |
| K02400 | X04076 |
| X07036 | X04408 |
| X07036 | X04408 |
| Y00816 | X05309 |
| N20475 | X05344 |
| M11233 | X05344 |
| X02544 | X05784 |
| X52192 | X06292 |
| R33755 | X06547 |
| X06989 | X06989 |
| X07979 | X07979 |
| X14723 | X08004 |
| M20566 | X12830 |
| M20566 | X12830 |
| X13197 | X13197 |
| M21304 | X13709 |
| X00351 | X13839 |
| X60236 | X14008 |
| H57180 | X14034 |
| M33011 | X14758 |
| X14768 | X14768 |
| M31625 | X14768 |
| M31626 | X14768 |
| M30816 | X14768 |
| X52882 | X14983 |
| AA598758 | X15187 |
| X15606 | X15606 |
| K03515 | X16539 |
| AA868186 | X17093 |
| X51416 | X51416 |
| M23699 | X51439 |
| AA455222 | X51675 |
| X51757 | X51757 |
| X52195 | X52195 |
| U06434 | X53682 |
| J03198 | X54048 |
| M32304 | X54533 |
| AA487812 | X56134 |
| X56134 | X56134 |
| M16985 | X56257 |
| N31660 | X56257 |
| H27379 | X57198 |
| X57522 | X57522 |
| X57830 | X57830 |
| M57765 | X58377 |
| X58528 | X58528 |
| M81182 | X58528 |
| S60489 | X60111 |
| A1739095 | X61157 |
| R76314 | X61587 |
| M83665 | X62534 |
| X65921 | X65921 |
| M37722 | X66945 |
| AA453816 | X69516 |
| AA187162 | X69654 |
| X69819 | X69711 |
| X70070 | X70070 |
| X70697 | X70697 |
| S40706 | X71427 |
| T53775 | X71874 |
| X71877 | X71877 |
| X73458 | X73458 |
| X74801 | X74801 |
| AA454585 | X75755 |
| R43734 | X76939 |
| Al189206 | X77303 |
| 2496221H1 | X77303 |
| H17504 | X80692 |
| R26434 | X80910 |
| X83688 | X83688 |
| U24231 | X84709 |
| 3576337H1 | X85030 |
| X87212 | X87212 |
| T56477 | X87212 |
| AA464034 | X89401 |
| X89576 | X89576 |
| AA187458 | X92396 |
| M15887 | X94565 |
| X94991 | X94991 |
| X96427 | X96427 |
| X97058 | X97058 |
| AA425120 | X98262 |
| 3283686H1 | XM_005825 |
| AW027188 | XM_005958 |
| 1525902F6 | XM_006646 |
| R48796 | XM_008099 |
| AK000599 | XM_027140 |
| AA044653 | XM_031608 |
| AW665954 | XM_035574 |
| H86407 | XM_037453 |
| R00285 | XM_038150 |
| X57447 | XM_039395 |
| 778372H1 | XM_040459 |
| R82530 | XM_041024 |
| A1580830 | XM_042041 |
| 3097063H1 | XM_044784 |
| 3038910H1 | XM_046691 |
| H53340 | XM_048213 |
| 1654210F6 | XM_048530 |
| L42856 | XM_054964 |
| 2707270F6 | XM_056259 |
| M23468 | Y00062 |
| Y00649 | Y00649 |
| Y00757 | Y00757 |
| M17017 | Y00787 |
| M28130 | Y00787 |
| L02932 | Y07619 |
| Y10256 | Y10256 |
| AA454813 | Y12395 |
| AA149850 | Y12670 |
| 704183H1 | Y13710 |
| 059476H1 | Y13829 |
| Y13834 | Y13834 |
| L11016 | Y14768 |
| 3141315H1 | Y17803 |
| AI341167 | Y18391 |
| AI707852 | Z12962 |
| U51278 | Z23115 |
| A1686653 | Z26876 |
| AA043102 | Z35102 |
| AA136533 | Z35481 |
| U49083 | Z49148 |
| R70234 | Z56852 |
| 257274R6 | Z58168 |
| U62027 | Z73157 |
| 391237F1 | Z73157 |
| 510997F1 | Z73157 |
| Al808621 | Z82214 |
| AA460801 | Z98749 |
| 2673259F6 | Z98752 |
| R22977 | Z98946 |
| L03380 | Z99995 |
| AA425422 | |
| AA460392 | |
| AA508510 | |
| AA552028 | |
| AA576785 | |
| AA663307 | |
| A1015248 | |
| A1024468 | |
| A1086865 | |
| A1190605 | |
| A1203269 | |
| A1264420 | |
| A1333013 | |
| A1435052 | |
| A1671268 | |
| A1796718 | |
| A1990816 | |
| AW027164 | |
| AW167520 | |
| H24679 | |
| H66015 | |
| H91370 | |
| W07570 | |
| 1274737F6 | |
| 195337H1 | |
| 2398102H1 | |
| 2531082H1 | |
| 264639H1 | |
| 2794246F6 | |
| 3290073H1 | |
| 335737H1 | |
| 4539942F8 | |
| 6300669H1 | |
| 938765H1 | |

**Table 2: Genes (by GenBank Accession Number) modulated at least 1.7 fold in primary AML Sample.**

| Gene ID | Accession No. |
|---|---|
| T94331 | AB026898 |
| 3881595H1 | AC000134 |
| 1329021 F6 | AC002073 |
| 2858615H1 | AC002325 |
| AI791539 | AC002428 |
| AI821217 | AC004258 |
| AA774798 | AC004671 |
| H29666 | AC004845 |
| T95173 | AC005071 |
| AA814523 | AC005160 |
| 5905620T9 | AC005212 |
| 5986963H1 | AC005280 |
| 5825251 H1 | AC005306 |
| N36113 | AC005670 |
| 1700438H1 | AC005682 |
| R48756 | AC005757 |
| 5538589F6 | AC005839 |
| 3918104H1 | AC006023 |
| AA443719 | AC007240 |
| AI867297 | AC007883 |
| R63067 | AC008073 |
| AW022174 | AC008382 |
| 5537789F6 | AC008525 |
| H00249 | AC008733 |
| 1436240H1 | AC008860 |
| 2668191 F6 | AC008949 |
| 5543360F8 | AC009220 |
| AA737674 | AC009892 |
| 5104579H1 | AC009892 |
| 3335217F6 | AC010311 |
| BE326380 | AC010521 |
| 3746214H1 | AC011088 |
| 1671315F6 | AC011500 |
| H60969 | AC012351 |
| AA926944 | AC012377 |
| 3100089H1 | AC012454 |
| Y00052 | AC013722 |
| R52476 | AC021078 |
| N45149 | AC021106 |
| A1742120 | AC022137 |
| 4177228F6 | AC022224 |
| 2676312H1 | AC022415 |
| H73476 | AC022740 |
| AA652121 | AC046170 |
| AI308320 | AC046170 |
| 1956982H1 | AC046170 |
| 1428534F6 | AC051619 |
| 2914934H1 | AC055707 |
| AA621370 | AC064807 |
| 5514511R6 | AC073333 |
| AI696737 | AC074331 |
| 3406131H1 | AC079118 |
| N20072 | AC096579 |
| 5911413H1 | AC096667 |
| AI458182 | AF042782 |
| 2291436H1 | AF074333 |
| W32067 | AF136745 |
| 6755801J1 | AF157623 |
| 2397317F6 | AF235100 |
| R53190 | AF384819 |
| 1731618H1 | AJ003147 |
| 2959801H1 | AJ003147 |
| 3123232H1 | AJ003147 |
| 2760110H1 | AJ006345 |
| X64073 | AJ239325 |
| 3986782F7 | AJ249275 |
| A1366098 | AJ276674 |
| A1695385 | AJ289236 |
| BE222911 | AJ400879 |
| AI400473 | AK017738 |
| AI299633 | AK021499 |
| R13802 | AK022901 |
| 1489075H1 | AK025775 |
| AI656222 | AL021155 |
| W96144 | AL021155 |
| 2459540H1 | AL031282 |
| 3461693F6 | AL031588 |
| 4333034H1 | AL031726 |
| 3332309H1 | AL031728 |
| R61661 | AL032821 |
| U71321 | AL033519 |
| AA935151 | AL034374 |
| AA010251 | AL034397 |
| U43431 | AL035367 |
| AA237071 | AL035420 |
| AA609779 | AL049610 |
| AA167461 | AL049612 |
| 4228729H2 | AL049742 |
| 6712339H1 | AL049766 |
| AI051176 | AL049872 |
| 1747028H1 | AL078600 |
| 5164454H1 | AL109840 |
| 7007735H1 | AL117382 |
| AA526337 | AL121601 |
| AI638342 | AL122004 |
| 4835576H1 | AL122035 |
| W01596 | AL133243 |
| U64205 | AL133367 |
| 4820983H1 | AL135786 |
| 5594552H1 | AL136381 |
| H12102 | AL136979 |
| H81171 | AL137073 |
| AA151374 | AL137790 |
| AA578089 | AL138787 |
| AL209040 | AL139082 |
| 6421806H1 | AL139396 |
| H60498 | AL157776 |
| 3721604H1 | AL160271 |
| H61066 | AL161787 |
| 2798009H1 | AL162252 |
| 2225447F6 | AL162430 |
| AI885557 | AL162729 |
| 2918417F6 | AL163279 |
| AA489975 | AL355151 |
| U77456 | AL355794 |
| 5375277T9 | AL356266 |
| AI051860 | AL356489 |
| 4019605F6 | AL356489 |
| U29607 | AL356801 |
| AA861429 | AL359512 |
| AA767859 | AL359915 |
| 1362587H1 | AL391122 |
| R09122 | AL391194 |
| R93094 | AP000173 |
| AA954331 | AP000432 |
| R10535 | AP000555 |
| 5327443H1 | AP000936 |
| 1569726H1 | AP001347 |
| R92422 | AP001672 |
| 3422674H1 | AP002800 |
| Al310451 | AP002812 |
| 3568042H1 | AP003900 |
| AA455969 | D00015 |
| AF030575 | D00015 |
| T64335 | D00017 |
| D12614 | D00102 |
| X67098 | D00596 |
| R27585 | D00759 |
| AA465593 | D00762 |
| M80436 | D10202 |
| M80436 | D10202 |
| M80436 | D10202 |
| M80436 | D10202 |
| AA464600 | D10493 |
| AI147046 | D10653 |
| S68252 | D11139 |
| M25315 | D12592 |
| AI186110 | D13118 |
| S57708 | D13515 |
| D13626 | D13626 |
| AA682625 | D13641 |
| AA598561 | D14043 |
| D14695 | D14695 |
| D14825 | D14825 |
| 855326R1 | D16234 |
| L20046 | D16305 |
| V00496 | D17206 |
| AA629808 | D17554 |
| M57285 | D21214 |
| J03801 | D21235 |
| A1700360 | D21878 |
| D25216 | D25216 |
| U41078 | D26512 |
| AF245447 | D28468 |
| AF245447 | D28468 |
| AA070997 | D29012 |
| 2134847H1 | D30756 |
| Al147295 | D30756 |
| AA455067 | D31839 |
| Al311090 | D38616 |
| AW629690 | D42084 |
| 1869911H1 | D42084 |
| 5122374H1 | D43701 |
| D43950 | D43950 |
| D45887 | D45887 |
| W68193 | D49489 |
| X72498 | D50326 |
| L11667 | D63861 |
| D63874 | D63874 |
| 3633286H1 | D63874 |
| X61598 | D83174 |
| AA279906 | D86550 |
| AA729988 | D86550 |
| D86956 | D86956 |
| L36719 | D87116 |
| D89078 | D89078 |
| U41070 | D89078 |
| Al821897 | D89675 |
| D90209 | D90209 |
| 2135769H1 | J02763 |
| J03040 | J03040 |
| 1876511H1 | J03072 |
| J03258 | J03258 |
| J03571 | J03571 |
| J04111 | J04111 |
| AI125073 | J04132 |
| 1634342H1 | J04794 |
| H30357 | J05451 |
| K02054 | K02054 |
| X02415 | K02569 |
| K03000 | K03000 |
| H58873 | K03195 |
| Al791949 | K03474 |
| L10413 | L00634 |
| H22919 | L03558 |
| L04288 | L04288 |
| AA405769 | L05144 |
| H62473 | L07594 |
| L08177 | L08177 |
| L08177 | L08177 |
| AA234097 | L08895 |
| AA464627 | L09604 |
| M94859 | L10284 |
| R78541 | L10717 |
| L15189 | L11086 |
| M15400 | L11910 |
| L12168 | L12168 |
| L12350 | L12350 |
| L12350 | L12350 |
| T87908 | L12387 |
| L09600 | L13974 |
| M14221 | L16510 |
| M60278 | L17032 |
| M60278 | L17032 |
| M60278 | L17032 |
| M60278 | L17032 |
| 2745317H1 | L17411 |
| M65128 | L18980 |
| W49672 | L20861 |
| A1380522 | L23822 |
| AA988469 | L25591 |
| NM_001168 | L26245 |
| R20939 | L31848 |
| 2470939H1 | L35848 |
| AA442810 | L36034 |
| L36148 | L36148 |
| M11723 | L43615 |
| M14745 | M14745 |
| W68291 | M15395 |
| M16038 | M16038 |
| 339598H1 | M16038 |
| M17783 | M17783 |
| 3171275H1 | M20199 |
| 5189380H1 | M21121 |
| 4130807F7 | M22440 |
| M22612 | M22612 |
| AA070627 | M22810 |
| 1445982H1 | M23254 |
| M28638 | M24906 |
| R45525 | M28215 |
| AI051962 | M28983 |
| 736837R6 | M29696 |
| M29870 | M29870 |
| Al264247 | M30309 |
| 1512407F6 | M30310 |
| M30471 | M30471 |
| M30704 | M30703 |
| AW467649 | M31158 |
| M84739 | M32294 |
| M84739 | M32294 |
| U52165 | M32315 |
| M35857 | M32315 |
| 5077322H1 | M32315 |
| N72918 | M34175 |
| M63193 | M58602 |
| M59465 | M59465 |
| 2294719H1 | M60858 |
| 2992331H1 | M63005 |
| AA069596 | M63582 |
| M63904 | M63904 |
| AI091579 | M63971 |
| M74782 | M74782 |
| AA410680 | M77016 |
| M80647 | M80647 |
| M84526 | M84526 |
| S93414 | M86553 |
| A1310138 | M91463 |
| M95678 | M95678 |
| 2017923F6 | M96326 |
| R60624 | NM_000702 |
| AA488324 | NM_001211 |
| AA488341 | NM_001336 |
| AF006823 | NM_002246 |
| 1322305T6 | NM_002250 |
| AI921879 | NM_002287 |
| AW129770 | NM_002349 |
| AJ004977 | NM_002873 |
| A1423526 | NM_003332 |
| 4516963H1 | NM_003576 |
| 3028719F6 | NM_003600 |
| AB010882 | NM_003601 |
| AF030424 | NM_003642 |
| AF029899 | NM_003814 |
| AF055993 | NM_003864 |
| A1220935 | NM_004142 |
| AW665782 | NM_004142 |
| AI191941 | NM_004226 |
| 1392516T6 | NM_004621 |
| AA449579 | NM_004769 |
| 1810447H1 | NM_004917 |
| AA047585 | NM_005109 |
| 4181072F6 | NM_005468 |
| AA448972 | NM_005592 |
| AA742351 | NM_005739 |
| 3406436F6 | NM_005845 |
| AJ001015 | NM_005854 |
| 3118530H1 | NM_005880 |
| AA906714 | NM_006573 |
| A]016020 | NM_006672 |
| AW770551 | NM_006770 |
| AW009940 | NM_006871 |
| 864164H1 | NM_007194 |
| 1467864F6 | NM_012089 |
| AF204944 | NM_012105 |
| W23427 | NM_012115 |
| 3363678H2 | NM_012226 |
| AI652076 | NM_012243 |
| 346874T6 | NM_013308 |
| Al522316 | NM_013386 |
| Al338030 | NM_013439 |
| A1700673 | NM_013439 |
| 4540025H1 | NM_014322 |
| W00842 | NM_014331 |
| AW511388 | NM_014358 |
| AW630208 | NM_014413 |
| H63640 | NM_014834 |
| AI743175 | NM_014959 |
| 2821685T6 | NM_014967 |
| W38474 | NM_015542 |
| AW243944 | NM_015596 |
| W07181 | NM_015701 |
| 2997457H1 | NM_015938 |
| AA631149 | NM_016205 |
| AA527334 | NM_016625 |
| 5543749F6 | NM_017414 |
| AW170305 | NM_017903 |
| AA160974 | NM_018155 |
| AA625433 | NM_018404 |
| AA074666 | NM_018834 |
| 767295H1 | NM_018983 |
| M78706 | NM_019020 |
| AF245447 | NM_020126 |
| AF245447 | NM_020126 |
| 4294821H1 | NM_020344 |
| 2490994H1 | NM_021624 |
| 3556218H1 | NM_021634 |
| 2435705R6 | NM_022048 |
| 3092423H1 | NM_022054 |
| W77977 | NM_022336 |
| AA429219 | NM_023930 |
| 1001514R6 | NM_024022 |
| AI031531 | NM_024083 |
| AA449040 | NM_024116 |
| 2803571H1 | NM_024586 |
| 1390130H1 | NM_024671 |
| 3241088H1 | NM_024850 |
| H96170 | NM_030779 |
| 1540906H1 | NM_030779 |
| AI824146 | NM_030811 |
| W90438 | NM_032127 |
| AA430653 | NM_032177 |
| 3495438F6 | NM_032294 |
| AW612141 | NM_033050 |
| AA417237 | NM_033225 |
| Al740538 | NM_033280 |
| M57703 | S63697 |
| 780099H1 | S63912 |
| AA714835 | S67156 |
| AA777347 | S76736 |
| AA521213 | S77359 |
| U39231 | S79852 |
| N77754 | S79873 |
| AA984230 | S80071 |
| U00672 | U00672 |
| U02478 | U02478 |
| AA019459 | U02680 |
| 3401107H1 | U03019 |
| AI580044 | U04816 |
| 3041874H1 | U07563 |
| 2457652H1 | U12465 |
| U39318 | U13175 |
| U13666 | U13666 |
| U13695 | U13695 |
| AA056652 | U14176 |
| AA456616 | U14970 |
| U18242 | U18242 |
| U18300 | U18300 |
| AA465444 | U18422 |
| U20537 | U20536 |
| U25128 | U25128 |
| U35237 | U26174 |
| A1884916 | U29171 |
| AA481076 | U31278 |
| NM_002411 | U33147 |
| 1671033F6 | U33429 |
| AA664389 | U35048 |
| 6313632H1 | U43030 |
| R09288 | U43522 |
| AA488645 | U47007 |
| U47077 | U47077 |
| 5801413H1 | U48449 |
| 2405358R6 | U48729 |
| AA186542 | U50078 |
| U51586 | U51586 |
| 1355140F1 | U51586 |
| AA455800 | U55206 |
| U56390 | U56390 |
| U83410 | U58088 |
| AA121261 | U58675 |
| AF027964 | U59911 |
| U60519 | U60519 |
| 2836805T6 | U62293 |
| U62433 | U62433 |
| 3188135H1 | U66673 |
| 3188135H1 | U66673 |
| 3188135H1 | U66673 |
| 3188135H1 | U66673 |
| 1360938T6 | U66679 |
| 809631T6 | U66684 |
| AA454652 | U67058 |
| A1214335 | U68755 |
| U69883 | U69883 |
| R98589 | U81375 |
| 5695322H1 | U82671 |
| AA745989 | U82979 |
| AA188256 | U83661 |
| 2526581H1 | U90904 |
| AA434064 | U95000 |
| AA773114 | U95740 |
| AA514978 | U96776 |
| Y07503 | V00510 |
| X96754 | V00557 |
| N67917 | V01512 |
| V01514 | V01514 |
| X87344 | X00369 |
| N53169 | X00567 |
| X02910 | X01394 |
| X01451 | X01451 |
| X01451 | X01451 |
| X01451 | X01451 |
| X01451 | X01451 |
| AA401046 | X02592 |
| 5537736F6 | X02592 |
| X87344 | X03066 |
| M10901 | X03225 |
| M54894 | X04403 |
| M54894 | X04403 |
| M54894 | X04403 |
| M54894 | X04403 |
| X07036 | X04408 |
| X07036 | X04408 |
| N75719 | X04744 |
| M19507 | X04876 |
| Y00816 | X05309 |
| M11233 | X05344 |
| AA479102 | X05972 |
| N24824 | X06182 |
| R33755 | X06547 |
| N41062 | X06820 |
| M86511 | X06882 |
| X07549 | X07549 |
| 1686702H1 | X07730 |
| X07979 | X07979 |
| X14723 | X08004 |
| J03561 | X12510 |
| J03561 | X12510 |
| J03561 | X12510 |
| J03561 | X12510 |
| M20566 | X12830 |
| M20566 | X12830 |
| M20566 | X12830 |
| M20566 | X12830 |
| U76549 | X12882 |
| M21304 | X13709 |
| X00351 | X13839 |
| X14830 | X14830 |
| X52882 | X14983 |
| AA598758 | X15187 |
| H27564 | X15729 |
| W15277 | X15940 |
| AA393214 | X15949 |
| M23502 | X16166 |
| K03515 | X16539 |
| M28880 | X16609 |
| 2403512H1 | X16674 |
| AA868186 | X17093 |
| J03236 | X51345 |
| X51416 | X51416 |
| AA411440 | X51521 |
| AA058828 | X51602 |
| AA455222 | X51675 |
| X51804 | X51804 |
| T72877 | X52015 |
| X52195 | X52195 |
| X52947 | X52947 |
| U06434 | X53682 |
| 3081284F6 | X53702 |
| M36821 | X53799 |
| AA490256 | X54048 |
| J03198 | X54048 |
| M60761 | X54228 |
| M11025 | X55283 |
| M33294 | X55313 |
| M33294 | X55313 |
| M31627 | X55543 |
| X55544 | X55544 |
| AA487812 | X56134 |
| X56134 | X56134 |
| X56777 | X56777 |
| H27379 | X57198 |
| M83652 | X57748 |
| X58528 | X58528 |
| M81182 | X58528 |
| S60489 | X60111 |
| X60592 | X60592 |
| R76314 | X61587 |
| M83665 | X62534 |
| R11490 | X62947 |
| A1436567 | X63422 |
| X63465 | X63465 |
| AA083577 | X63527 |
| X63547 | X63546 |
| 2159360H1 | X63692 |
| X64074 | X63926 |
| X63926 | X63926 |
| X64083 | X63926 |
| 2535659H1 | X69168 |
| AA187162 | X69654 |
| X69819 | X69711 |
| AI310990 | X71491 |
| T53775 | X71874 |
| 3285272H1 | X73568 |
| U11087 | X75299 |
| X75299 | X75299 |
| AA454585 | X75755 |
| X75821 | X75821 |
| X75918 | X75918 |
| X76029 | X76029 |
| R43734 | X76939 |
| A1189206 | X77303 |
| H17504 | X80692 |
| R26434 | X80910 |
| AI521155 | X81892 |
| AA088861 | X83228 |
| U10440 | X84849 |
| 407169H1 | X84909 |
| 3576337H1 | X85030 |
| T55802 | X85117 |
| 4407508H1 | X85337 |
| AA025432 | X85373 |
| T56477 | X87212 |
| AA464034 | X89401 |
| X89576 | X89576 |
| X89576 | X89576 |
| R83270 | X89750 |
| 917064H1 | X91249 |
| X91809 | X91809 |
| X92106 | X92106 |
| AA187458 | X92396 |
| AJ000519 | X92962 |
| X94991 | X94991 |
| X96427 | X96427 |
| R85213 | X98022 |
| X98296 | X98296 |
| X99585 | X99585 |
| R48796 | XM_008099 |
| R50354 | XM_009915 |
| W15172 | XM_016514 |
| AK000599 | XM_027140 |
| 7157414H1 | XM_031246 |
| AA044653 | XM_031608 |
| L16953 | XM_032556 |
| 1266202T6 | XM_033674 |
| AA805691 | XM_033788 |
| AA861582 | XM_036492 |
| H86407 | XM_037453 |
| 778372H1 | XM_040459 |
| AA016239 | XM_041087 |
| A1580830 | XM_042041 |
| Al732875 | XM_042637 |
| AA463411 | XM_045320 |
| AA648280 | XM_046411 |
| 3038910H1 | XM_046691 |
| H63831 | XM_047328 |
| 1654210F6 | XM_048530 |
| AA460131 | XM_049228 |
| 5539620F6 | XM_049755 |
| AA682896 | XM_050250 |
| L42856 | XM_054964 |
| 1483347H1 | XM_056259 |
| AI307255 | XM_058135 |
| H74265 | Y00062 |
| Y00064 | Y00064 |
| M17017 | Y00787 |
| M28130 - | Y00787 |
| L02932 | Y07619 |
| AA504415 | Y09781 |
| AI809636 | Y12336 |
| AA516206 | Y12851 |
| 000527H1 | Y13829 |
| 059476H1 | Y13829 |
| Y13834 | Y13834 |
| L11016 | Y14768 |
| 3141315H1 | Y17803 |
| 551234R6 | Y17803 |
| AA426103 | Y18000 |
| H97778 | Z13009 |
| AA402431 | Z15005 |
| L07555 | Z22576 |
| U51278 | Z23115 |
| M58525 | Z26491 |
| AW772610 | Z26652 |
| Z29090 | Z29090 |
| H19371 | Z32684 |
| AA136533 | Z35481 |
| Z48810 | Z48810 |
| U49083 | Z49148 |
| R70234 | Z56852 |
| 4902714H1 | Z69918 |
| 150224T6 | Z80147 |
| M29871 | Z82188 |
| A1808621 | Z82214 |
| AA699919 | Z83821 |
| 5538394H1 | Z83843 |
| 5020377F9 | Z97832 |
| AA460801 | Z98749 |
| A1625585 | Z98750 |
| 2673259F6 | Z98752 |
| R22977 | Z98946 |
| AA007595 | |
| AA188574 | |
| AA280754 | |
| AA283874 | |
| AA460392 | |
| AA508510 | |
| AA515469 | |
| AA526772 | |
| AA576785 | |
| AA634241 | |
| AA663307 | |
| AA663482 | |
| AA713864 | |
| AA714520 | |
| AA828809 | |
| AA868502 | |
| AI061445 | |
| AI086865 | |
| AI264420 | |
| AI378131 | |
| AI440504 | |
| AI567491 | |
| AI693066 | |
| A1709066 | |
| A1766478 | |
| Al821337 | |
| A1949694 | |
| AW439329 | |
| AW630054 | |
| H24679 | |
| H29257 | |
| H51856 | |
| H66015 | |
| H72339 | |
| N57580 | |
| N54592 | |
| WO7570 | |
| T75463 | |
| R88730 | |
| R91509 | |
| T56441 | |
| T77711 | |
| W92423 | |
| 1274737F6 | |
| 1338107F6 | |
| 1508571F6 | |
| 1548205H1 | |
| 1594182F6 | |
| 1594701F6 | |
| 1879290H1 | |
| 1902928H1 | |
| 194370H1 | |
| 195337H1 | |
| 198381H1 | |
| 2021568H1 | |
| 205203T6 | |
| 2194064H1 | |
| 224922R6 | |
| 2398102H1 | |
| 2531082H1 | |
| 2630745F6 | |
| 264639H1 | |
| 2704982H1 | |
| 2716787H1 | |
| 2798810F6 | |
| 2832401 H1 | |
| 2894096F6 | |
| 2919406F6 | |
| 2937644F6 | |
| 2950021H1 | |
| 3010621F6 | |
| 3123948H1 | |
| 3253054R6 | |
| 3290073H1 | |
| 3330472H1 | |
| 335737H1 | |
| 3674358H1 | |
| 3749346F6 | |
| 3820429H1 | |
| 3978404F6 | |
| 4031124H1 | |
| 4056384H1 | |
| 4097060H1 | |
| 4288779H1 | |
| 4301823H1 | |
| 4558488F6 | |
| 4570377H1 | |
| 5058893F9 | |
| 5541621H1 | |
| 5546249F6 | |
| 5546336H1 | |
| 5771839H1 | |
| 5804485H1 | |
| 5849807H1 | |
| 6530555H1 | |
| 656258H1 | |
| 6591535H1 | |
| 859993H1 | |
| 930273R6 | |
| 938765H1 | |

**Table 3: Genes (By Genbank Accession Number) modulated at least 1.5 fold in all cell lines and at least 1.7 fold in patient responder sample.**

| Gene ID | Accession No. |
|---|---|
| 5543360F8 | AC009220 |
| AA237071 | AL035420 |
| AA455969 | D00015 |
| M25315 | D12592 |
| U41078 | D26512 |
| L10413 | L00634 |
| AA464627 | L09604 |
| 2470939H1 | L35848 |
| M84526 | M84526 |
| Al921879 | NM_002287 |
| AF204944 | NM_012105 |
| W77977 | NM_022336 |
| AA449040 | NM_024116 |
| AA521213 | S77359 |
| AA984230 | S80071 |
| AA456616 | U14970 |
| AI884916 | U29171 |
| U60519 | U60519 |
| X00351 | X13839 |
| AA868186 | X17093 |
| H27379 | X57198 |
| AA454585 | X75755 |
| X89576 | X89576 |
| AI580830 | XM_042041 |
| U49083 | Z49148 |
| 2398102H1 | |
| 2531082H1 | |

## Claims

1. A method of determining wheter a patient with acute myelogenous leukemia (AML) will respond to treatment with a farnesyl transferase inhibitor (FTI) by:
(a) analysing a diseased cell from a bone marrow sample from the patient for a detectable difference in the amount of expression of a gene comprising SEQ ID NO: 846 (3434105F7) following treatment with a FTI relative to an untreated diseased cell baseline;
(b) comparing the detectable difference from step (a) to those obtained from responder and non responder patients; and
(c) identifying whether the patient has a responder or non-responder expression pattern to determine whether treatment of the patient with a FTI could be indicated.

2. A method of monitoring treatment response in a patient with acute myelogenous leukemia treated with a FTI by:
(a) analysing a diseased cell from a bone marrow sample from the patient for a detectable difference in the amount of expression of a gene comprising SEQ ID NO: 846 (3434105F7) at various periods throughout the course of treatment;
(b) comparing the expression pattern from step (a) to those obtained from responder and non-responder patients; and
(c) identifying whether the patient has a responder or non-responder expression pattern to determine whether treatment of the patient is continued.

3. The method of claim 1 or claim 2, wherein the diseased cells are hematopoietic blast cells.

4. The method of any preceding claim, wherein step (a) involves analysing the amount of expression of a combination consisting of the genes shown in: (i) Tables 1-3 or (ii) Table 1.

5. A method according to any preceding claim, wherein the analysis of step (a) is carried out using a nucleic acid array.

6. The method of claim 5 wherein the detectable difference is at least 1.5 fold compared to the baseline.

7. The method of claim 6, wherein the detectable difference is at least 1.7 fold.

8. The method of claim 7, wherein the detectable difference is at least 2 fold.

9. The method according to any previous claim wherein the FTI is a quinoline or a quinoline derivative.

10. The method of claim 9 wherein the FTI is:
7-(3-chlorophenyl)-9-[(4-chlorophenyl)-1H-imidazol-1-ylmethyl]-2,3-dihydro-1H,5H-benzo[ij]quinolizin-5-one;
7-(3-chlorophenyl)-9-[(4-chlorophenyl)-1H-imidazol-1-ylmethyl]-1,2-dihydro-4H-pyrrolo[3,2,1-ij]quinoline-4-one;
8-[amino(4-chlorophenyl)(1-methyl-1H-imidazol-5-yl)methyl]-6-(3-chlorophenyl)-1,2-dihydro-4H-pyrrolo[3,2,1-ij]quinolin-4-one;
8-[amino(4-chlorophenyl)(1-methyl-1H-imidazol-5-yl)methyl]-6-(3-chlorophenyl)-2,3-dihydro-1H,5H-benzo[ij]quinolizin-5-one; or
(B)-6-[amino(4-chlorophenyl)(1-methyl-1H-imidazol-5-yl)methy]-4-(3-chlorophenyl)-1-methyl-2(1 H)-quinolinone).

11. The method of claim 10 wherein the FTI is (B)-6-[amino(4-chlorophenyl)(1-methyl- 1H-imidazol-5-yl)methyl]-4-(3-chlorophenyl)-1-methyl-2(1H)-quinolinone).

12. Use of an FTI in the manufacture of a medicament for the treatment of an AML patient, where the patient has been determined to be a responder by the method of any one of claims 1 to 11.

13. The use of claim 12, wherein the medicament comprises another therapeutic composition.

14. The use of claim 13, wherein said another therapeutic composition modulates an MAPK/ERK signalling pathway, TGFβ, WNT or an apoptotic pathway.

15. The use of claim 14, wherein said another composition is a tyrosine kinase inhibitor, MEK kinase inhibitor, PI3 kinase inhibitor, MAP kinase inhibitor, apoptosis modulator, or a combination thereof.

## Patentansprüche

1. Verfahren zur Bestimmung, ob ein Patient mit akuter myelogener Leukämie (AML) auf eine Behandlung mit einem Farnesyltransferaseinhibitor (FTI) ansprechen wird, mittels:
(a) Überprüfung einer kranken Zelle aus einer Knochenmarksprobe des Patienten auf eine nachweisbare Differenz in der Höhe der Expression eines Gens, umfassend SEQ ID NO: 846 (3434195F7) nach Behandlung mit einem FTI im Vergleich mit einer unbehandelten kranken Zelle als Bezugswert,
(b) Vergleich der nachweisbaren Differenz aus Punkt (a) mit solchen, die bei auf die Behandlung ansprechenden und nicht-ansprechenden Patienten festgestellt wurden, und
(c) Bestimmung, ob der Patient ein Genexpressionsmuster eines auf die Behandlung ansprechenden oder nicht ansprechenden Patienten aufweist, um festzustellen, ob die Behandlung des Patienten mit einem FTI indiziert sein kann.

2. Verfahren zur Beobachtung der Behandlungsreaktion eines Patienten mit akuter myelogener Leukämie, der mit einem FTI behandelt wurde, mittels:
(a) Überprüfung einer kranken Zelle aus einer Knochenmarksprobe des Patienten auf eine nachweisbare Differenz in der Höhe der Expression eines Gens, umfassend SEQ ID NO: 846 (3434105F7) zu verschiedenen Zeitpunkten während des Behandlungsverlaufs,
(b) Vergleich des Genexpressionsmusters aus Schritt (a) mit solchen, die bei auf die Behandlung ansprechenden und nicht-ansprechenden Patienten festgestellt wurden, und
(c) Bestimmung, ob der Patient ein Genexpressionsmuster eines auf die Behandlung ansprechenden oder nicht-ansprechenden Patienten aufweist, um festzustellen, ob die Behandlung des Patienten mit einem FTI fortgesetzt werden soll.

3. Verfahren nach Anspruch 1 oder Anspruch 2, wobei die kranken Zellen hämatopoietische Blastenzellen sind.

4. Verfahren nach einem der vorangehenden Ansprüche, wobei Schritt (a) die Analyse der Expressionshöhe einer Kombination von Genen beinhaltet, die in (i) Tabellen 1-3 oder (ii) Tabelle 1 verzeichnet sind.

5. Verfahren nach einem der vorhergehenden Ansprüche, wobei die Analyse von Schritt (a) mit Hilfe eines Nukleinsäureassays durchgeführt wird.

6. Verfahren nach Anspruch 5, wobei die nachweisbare Differenz mindestens das 1,5-fache im Vergleich zum Bezugswert beträgt.

7. Verfahren nach Anspruch 6, wobei die nachweisbare Differenz mindestens das 1,7-fache beträgt.

8. Verfahren nach Anspruch 7, wobei die nachweisbare Differenz mindestens das 2-fache beträgt.

9. Verfahren nach einem der vorangehenden Ansprüche, wobei es sich bei dem FTI um ein Chinolin oder ein Chinolinderivat handelt.

10. Verfahren nach Anspruch 9, wobei es sich bei dem FTI um eine der folgenden Verbindungen handelt:
7-(3-Chlorphenyl)-9-[(4-chlorphenyl)-1H-imidazol-1-ylmethyl]-2,3-dihydro-1H,5H-benz[ij]chinolizin-5-on;
7-(3-Chlorphenyl)-9-[(4-chlorphenyl)-1H-imidazol-1-ylmethyl]-1,2-dihydro-4H-pyrrolo[3,2, 1-ij]chinolin-4-on;
8-[Amino(4-chlorphenyl)(1-methyl-1 H-imidazol-5-yl)methyl]-6-(3-chlorphenyl)-1,2-dihydro-4H-pyrrolo[3,2,1-ij]chinolin-4-on;
8-[Amino(4-chlorphenyl)(1 -methyl-1H-imidazol-5-yl)methyl]-6-(3-chlorphenyl)-2,3-dihydro-1H,5H-benz[ij]chinolizin-5-on; oder
(B)-6-[Amino(4-chlorphenyl)(1-methyl-1H-imidazol-5-yl)methyl]-4-(3-chlorphenyl)-1-methyl-2(1H)-chinolinon).

11. Verfahren nach Anspruch 10, wobei es sich bei dem FTI um (B)-6-[Amino(4-chlorphenyl)(1-methyl-1H-imidazol-5-yl)methyl]-4-(3-chlorphenyl)-1-methyl-2(1H)-chinolinon) handelt.

12. Verwendung eines FTI zur Herstellung eines Medikaments zur Behandlung eines an AML erkrankten Patienten, wobei der Patient mit dem Verfahren nach einem der Ansprüche 1- 11 als ein auf die Behandlung ansprechendes Individuum identifiziert wurde.

13. Verwendung nach Anspruch 12, wobei das Medikament eine andere therapeutische Zusammensetzung beinhaltet.

14. Verwendung nach Anspruch 13, wobei die besagte andere therapeutische Zusammensetzung einen MAPK/ERK-Signalweg, TGFβ-, WNT- oder einen apoptotischen Signalweg beeinflußt.

15. Verwendung nach Anspruch 14, wobei es sich bei der besagten anderen Zusammensetzung um einen Tyrosinkinaseinhibitor, einen MEK-Kinaseinhibitor, einen PI3-Kinaseinhibitor, einen MAP-Kinaseinhibitor, einen Apoptosemodulator oder eine Kombination davon handelt.

## Revendications

1. Méthode permettant de déterminer si un patient atteint d'une leucémie myéloïde aiguë (AML) répondra à un traitement avec un inhibiteur d'une farnésyl transférase (FTI) par ;
(a) l'analyse d'une cellule malade provenant d'un échantillon de moelle osseuse du patient pour déterminer une différence détectable dans la quantité d'expression d'un gène comprenant la séquence SEQ ID NO : 846 (3434105F7) après un traitement avec un FTI par rapport à une ligne de base correspondant à une cellule malade non-traitée ;
(b) la comparaison de la différence détectable de l'étape (a) à celles obtenues auprès de patients répondants et non-répondants ; et
(c) le fait d'identifier si le patient a un profil d'expression de type répondant ou non-répondant pour déterminer si le traitement du patient avec un FTI pourrait être indiqué.

2. Méthode de contrôle d'une réponse à un traitement chez un patient atteint d'une leucémie myéloïde aiguë traité avec un FTI par :
(a) l'analyse d'une cellule malade provenant d'un échantillon de moelle osseuse du patient pour déterminer une différence détectable dans la quantité d'expression d'un gène comprenant la séquence SEQ ID NO : 846 (3434105F7) à différents moments tout au long du traitement ;
(b) la comparaison du profil d'expression de l'étape (a) à ceux obtenus auprès de patients répondants et non-répondants ; et
(c) le fait d'identifier si le patient a un profil d'expression de type répondant ou non-répondant pour déterminer si le traitement du patient est poursuivi.

3. Méthode selon la revendication 1 ou la revendication 2, dans laquelle les cellules malades sont des cellules blastiques hématopoïétiques.

4. Méthode selon l'une quelconque des revendications précédentes, dans laquelle l'étape (a) implique l'analyse de la quantité d'expression d'une combinaison constituée des gènes présentés dans : (i) les tableaux 1 à 3 ou (ii) le tableau 1.

5. Méthode selon l'une quelconque des revendications précédentes, dans laquelle l'analyse de l'étape (a) est réalisée en utilisant un réseau d'acides nucléiques.

6. Méthode selon la revendication 5, dans laquelle la différence détectable est d'au moins 1,5 fois par rapport à la ligne de base.

7. Méthode selon la revendication 6, dans laquelle la différence détectable est d'au moins 1,7 fois.

8. Méthode selon la revendication 7, dans laquelle la différence détectable est d'au moins 2 fois.

9. Méthode selon l'une quelconque des revendications précédentes, dans laquelle le FTI est une quinoline ou un dérivé de quinoline.

10. Méthode selon la revendication 9, dans laquelle le FTI est :
la 7-(3-chlorophényl)-9-[(4-chlorophényl)-1H-imidazol-1-ylméthyl]-2,3-dihydro-1H,5H-benzo[ij]-quinolizin-5-one ;
la 7-(3-chlorophényl)-9-[(4-chlorophényl)-1H-imidazol-1-ylméthyl]-1,2-dihydro-4H-pyrrolo[3,2,1-ij]-quinoline-4-one ;
la 8-[amino(4-chlorophényl)(1-méthyl-1H-imidazol-5-yl)méthyl]-6-(3-chlorophényl)-1,2-dihydro-4H-pyrrolo-[3,2,1-ij]-quinolin-4-one ;
la 8-[amino(4-chlorophényl)(1-méthyl-1H-imidazol-5-yl)méthyl]-6-(3-chlorophényl)-2,3-dihydro-1H,5H-benzo[ij]-quinolizin-5-one ; ou
la (B)-6-[amino(4-chlorophényl)(1-méthyl-1H-imidazol-5-yl)méthyl]-4-(3-chlorophényl)-1-méthyl-2(1H)-quinolinone.

11. Méthode selon la revendication 10, dans laquelle le FTI est la (B)-6-[amino(4-chlorophényl) (1-méthyl-1H-imidazol-5-yl)méthyl]-4-(3-chlorophényl)-1-méthyl-2(1)-quinolinone.

12. Utilisation d'un FTI dans la fabrication d'un médicament destiné au traitement d'un patient atteint d'une AML, où il a été déterminé que le patient est un répondant d'après la méthode selon l'une quelconque des revendications 1 à 11.

13. Utilisation selon la revendication 12, dans laquelle le médicament comprend une autre composition thérapeutique.

14. Utilisation selon la revendication 13, dans laquelle ladite autre composition thérapeutique module une voie de signalisation MAPK/ERK, TGFβ, WNT ou une voie apoptotique.

15. Utilisation selon la revendication 14, dans laquelle ladite autre composition est un inhibiteur de tyrosine kinase, un inhibiteur de MEK kinase, un inhibiteur de PI3 kinase, un inhibiteur de MAP kinase, un modulateur d'apoptose ou une combinaison de ceux-ci.
